# EUROPEAN PATENT APPLICATION

(11) **EP 3 130 377 A1**
(43) Date of publication of application: **15.02.2017**
(21) Application number: 15789982.4
(22) Date of filing: 07.05.2015
(51) Int. Cl.: A63B 21/00, A63B 23/035, A63B 23/12

(54) **MUSCLE TRAINING METHOD**

(30) Priority: 09.05.2014 JP 2014098078
(71) Applicant: Kaatsu Japan Co., Ltd., Tokyo 183-0016 (JP)
(72) Inventor: SATO, Yoshiaki, Fuchu-shi Tokyo 183-0016 (JP)
(74) Representative: Gill Jennings & Every LLP
(86) International application number: PCT/JP2015/063229
(87) International publication number: WO 2015/170719

(57) **Abstract**

Provided is a method that is capable of strengthening muscles very effectively. The method is a muscle training method including: asking a user to perform underwater exercise while winding a belt around at least one of four limbs of the user and applying specific pressure to restrict blood circulation at a muscle of the user without stopping the blood circulation. The blood circulation of the muscle of the user is appropriately restricted so as to produce growth hormones more and additionally a load can be given effectively from motion less than on the ground, and from the synergic action of them, muscles can be strengthened very easily.

## Description

### Technical Field

The present invention relates to muscle training methods.

### Background Art

Conventionally a KAATSU muscle training method has been proposed, which is capable of strengthening muscles effectively by applying load to muscles while appropriately restricting blood circulation thereto, and such a method has been put to practice use (see Patent Document 1, for example). In such a muscle training method, a muscle strengthening tool is used, which is configured to apply pressure to a muscle while tightening a predetermined part of four limbs (arms and legs) of a user with a tightening tool.

### Citation List

### Patent Document

Patent Document 1: JP 2670421

### Summary

### Problem to be Solved

Meanwhile a method relating to training or rehabilitation using a resisting force or a buoyancy force acting in water has been proposed. There is an underwater training method that athletes are currently employing, such as a method of swimming while attaching an object (e.g., a bucket) via a rope to the body of an athlete to generate a large resisting force in water, or a method of swimming while against water current. According to such a method, a load can be put on a user (a person who gets training) intentionally.

These conventional underwater training methods, however, have a problem that the level of lactic acid in the blood of the user cannot increase effectively considering a required large tool or such equipment. In order to strengthen muscles, growth hormones have to be produced more than usual from the pituitary gland, and to this end, the level of lactic acid in the blood has to be increased effectively. The inventor of the present application newly found the ability of strengthening muscles very effectively by combining a conventional KAATSU muscle training method as described in Patent Document 1 as stated above with underwater training.

In water, a user can move the body relatively easily because of a buoyancy force acting. Although this can provide the environment for an obese person or a person with the muscle strength declined where they can easily conduct training or rehabilitation, a sufficient load cannot be given to the user because the buoyancy force acts, and so there is another problem of the difficulty to achieve the effect from the training or rehabilitation. The inventor of the present application found that such a problem also can be solved by the combination of a conventional KAATSU muscle training method with underwater training.

The present invention aims to provide a method that is capable of strengthening muscles very effectively.

### Means for Solving Problem

To fulfill the above aim, a method according to the present invention is for training of a muscle of a user, and includes: asking the user to perform underwater exercise while winding a belt around at least one of four limbs of the user and applying specific pressure to restrict blood circulation at a muscle of the user without stopping the blood circulation.

Such a method which includes winding of the belt around at least one of four limbs of a user to apply specific pressure to a muscle of the user to restrict the blood circulation there without stopping it allows growth hormones of the user to be produced more. Further, a user who conducts such underwater exercise while restricting the blood circulation appropriately can receive a load that cannot be received on the ground. That is, the blood circulation of the muscle of the user is appropriately restricted, whereby the level of lactic acid in the blood of the user can be increased to produce growth hormones more without the need of employing a large tool or such equipment as in the conventional underwater training method. Additionally a load can be given effectively from motion less than on the ground, and from the synergic action of them, muscles can be strengthened very easily. Moreover, since a buoyancy force also can be obtained in water, a user who has difficulty in supporting their weight because of muscle strength declined also can strengthen their muscles using this method.

In the muscle training method according to the present invention, the belt may be wound around a base of an arm of the user and specific pressure may be applied thereto to restrict blood circulation at a muscle of the arm of the user without stopping the blood circulation for arm pressurization. At this time, the specific pressure may be set in a range of 80 to 120 mmHg. Further, while receiving the arm pressurization, the user may be asked to perform specific swim exercise including an arm/leg propulsion exercise to move forward by making strokes with arms and by kicking water, and an arm propulsion exercise to move forward by making strokes with the arms only.

The specific swim exercise may include: a first step of performing the arm/leg propulsion exercise of 200 yards continuously; a second step of performing the arm/leg propulsion exercise of 50 yards four times while having a specific stop duration therebetween; and a third step of performing the arm propulsion exercise of 50 yards, followed by the arm/leg propulsion exercise of 50 yards while having a specific stop duration therebetween. The arm/leg propulsion exercise in the second step may be performed with intensity higher than in the arm/leg propulsion exercise in the first step. The arm/leg propulsion exercise in the third step may be performed with intensity higher than in the arm/leg propulsion exercise in the second step.

The specific swim exercise may include: a first step of performing the arm/leg propulsion exercise of 200 yards continuously; a fourth step of performing the arm propulsion exercise of 75 yards, followed by the arm/leg propulsion exercise of 75 yards; and a fifth step of performing the arm/leg propulsion exercise of 25 yards four times while having a specific stop duration therebetween. The arm/leg propulsion exercise in the fourth step is performed with intensity higher than in the arm/leg propulsion exercise in the first step. The arm/leg propulsion exercise in the fifth step is performed with intensity equal to intensity of the arm/leg propulsion exercise in the fourth step.

In the muscle training method according to the present invention, the user may be asked to perform walking exercise in water while setting the specific pressure for the arm pressurization at a value exceeding 120 mmHg.

In the muscle training method according to the present invention, the belt may be wound around a base of a leg of the user and specific pressure may be applied thereto to restrict blood circulation at a muscle of the leg of the user without stopping the blood circulation for leg pressurization. At this time, the specific pressure may be set in a range of 100 to 140 mmHg. While receiving the leg pressurization, the user may be asked to perform specific swim exercise including an arm/leg propulsion exercise to move forward by making strokes with arms and by kicking water, and a leg propulsion exercise to move forward by kicking water only.

The specific swim exercise may include: a sixth step of performing the arm/leg propulsion exercise of 25 yards four times while having a specific stop duration therebetween; and a seventh step of performing the leg propulsion exercise of 50 yards, followed by the arm/leg propulsion exercise of 50 yards while having a specific stop duration therebetween. At this time, the seventh step may be repeated twice.

The specific swim exercise may include: an eighth step of performing the leg propulsion exercise of 75 yards, followed by the arm/leg propulsion exercise of 75 yards while having a specific stop duration therebetween; and a ninth step of performing the arm/leg propulsion exercise of 25 yards four times while having a specific stop duration therebetween.

In the muscle training method according to the present invention, the user may be asked to perform walking exercise in water while setting the specific pressure for the leg pressurization at a value exceeding 140 mmHg.

The muscle training method according to the present invention may include: winding the belt around a base of an arm of the user and applying specific pressure thereto to restrict blood circulation at a muscle of the arm of the user without stopping the blood circulation for arm pressurization; and winding the belt around a base of a leg of the user and applying specific pressure thereto to restrict blood circulation at a muscle of the leg of the user without stopping the blood circulation for leg pressurization, which is performed after the arm pressurization. At this time, the specific pressure for the arm pressurization may be set in a range of 80 to 120 mmHg, and the specific pressure for the leg pressurization may be set in a range of 100 to 140 mmHg. In the method, while receiving the arm pressurization, the user may be asked to perform specific swim exercise including an arm/leg propulsion exercise to move forward by making strokes with arms and by kicking water, and an arm propulsion exercise to move forward by making strokes with the arms only, and while receiving the leg pressurization, the user may be asked to perform specific swim exercise including the arm/leg propulsion exercise, and a leg propulsion exercise to move forward by kicking water only.

In the muscle training method according to the present invention, while the specific pressure for the arm pressurization is set at a value exceeding 120 mmHg and the specific pressure for the leg pressurization is set at a value exceeding 140 mmHg, the user may be asked to perform walking exercise in water.

### Advantageous Effect of Invention

The present invention can provide a method that is capable of strengthening muscles very effectively.

### Brief Description of Drawings

FIG. 1 is a front view of a belt used in a muscle training method according to one embodiment of the present invention.
FIG. 2 is a rear view of the belt of FIG. 1.
FIG. 3 is a plan view of the belt of FIG. 1.
FIG. 4 is a bottom view of the belt of FIG. 1.
FIG. 5 is a right side view of the belt of FIG. 1.
FIG. 6 is a left side view of the belt of FIG. 1.
FIG. 7 is a cross-sectional view taken along A-A of FIG. 1.
FIG. 8 shows the belt of FIG. 1 in use.
FIG. 9 is a flowchart to describe a muscle training method according to one embodiment of the present invention.

### Description of Embodiments

The following describes one embodiment of the present invention, with reference to the drawings.

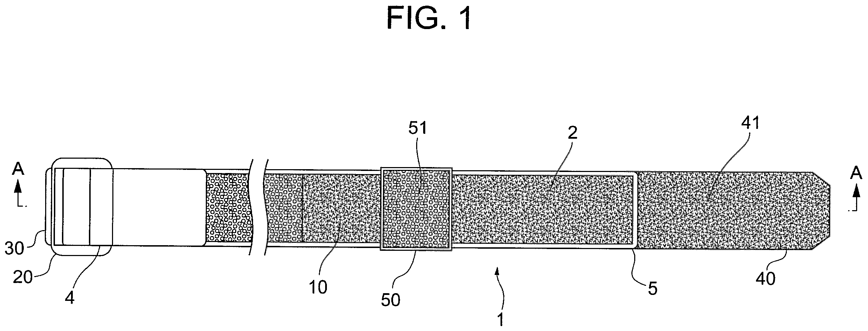

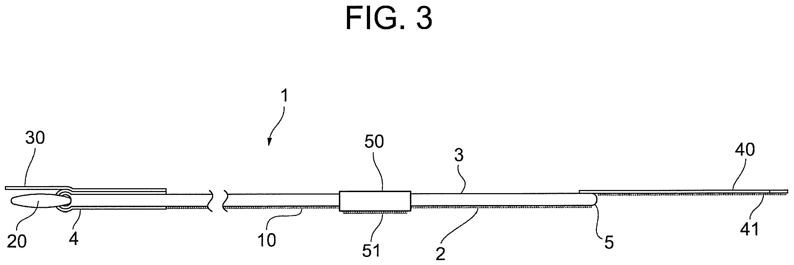

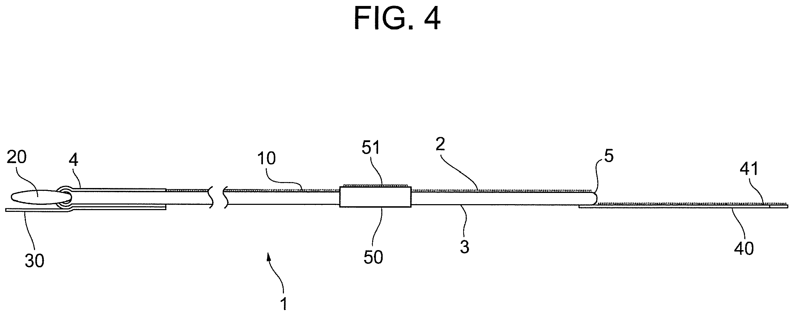

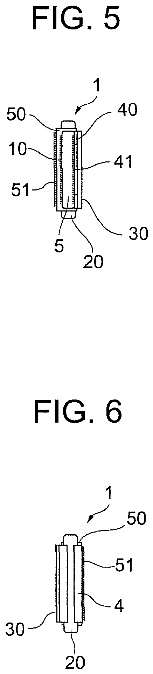

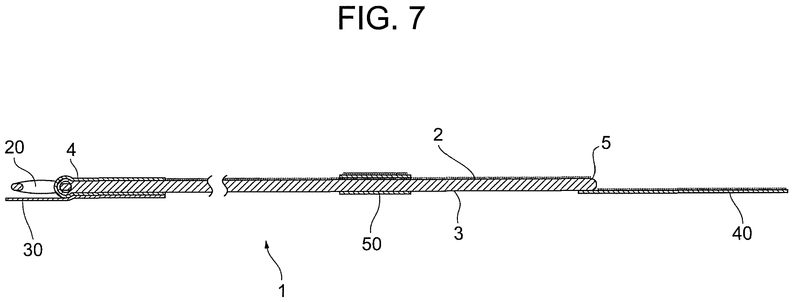

Referring firstly to FIGs. 1 through 8, a belt 1 to be used in a muscle training method according to one embodiment of the present invention (hereinafter called "KAATSU underwater training") is described.

The belt 1 in the present embodiment is a belt-like member that is wound around at least one of four limbs of a user to apply appropriate specific pressure to a muscle of the user to restrict the blood circulation of the muscle without stopping it, and is made of a material having water repellency, water resistance, and a quick-drying property as well as elasticity (e.g., neoprene rubber). The belt 1 in the present embodiment is therefore suitable for exercise in water.

The length or the width of the belt 1 can be appropriately set depending on the physique of the user or a body part for winding. A plurality of belts 1 (specifically four for pressurization to both arms and both legs of a person who conducts KAATSU underwater training) can be used. The number of the belts 1 is not always four, which may be any number that is one or more. The belts 1 in the same number do not have to be used for arms and for legs.

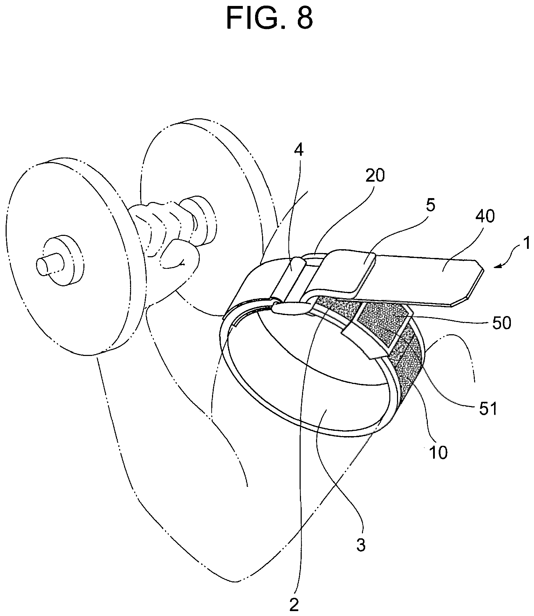

As shown in FIGs. 1 and 8, the belt 1 is provided with a hook-and-loop fastener 10 on the outer face (exposed to the outside) 2. The hook-and-loop fastener 10 is to keep a loop shape of the belt 1 in the state where pressure is applied to a muscle. The hook-and-loop fastener 10 may be provided at an appropriate position depending on the length or the like of the belt 1. The hook-and-loop fastener 10 may be provided on the inner face 3 of the belt 1.

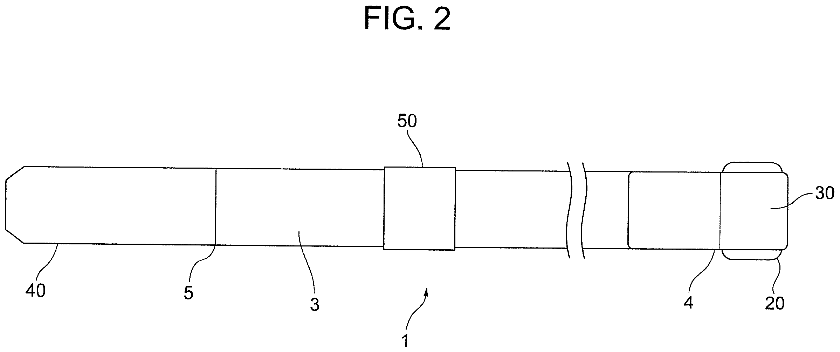

The belt 1 has one end 4, to which a buckle 20 is attached. The buckle 20 is to allow the other end 5 of the belt 1 to pass therethrough during winding-around of the belt and then to allow the belt 1 to be folded back. As shown in FIGs. 2 through 4, the buckle 20 has a small piece 30 made of a soft material (e.g. leather or cloth) on the inner face (the side facing the skin of the user). This small piece 30 prevents, when the belt 1 is tightened to apply pressure, the skin of the user (together with a part of the belt 1 passing through the buckle 20) from getting caught in the buckle 20.

A belt-like member 40 is connected to the belt 1 at the end 5 on the opposite side of the buckle 20, the belt-like member having the same width as the width of the belt 1 (or the width slightly narrower than the width of the belt 1). The belt-like member 40 is provided with a hook-and-loop fastener 41 on the outer face (exposed to the outside), and this hook-and-loop fastener 41 is configured to couple with a hook-and-loop fastener 51 of an annular member 50 provided at a center part of the belt 1. These belt-like member 40 and annular member 50 are coupled via their hook-and-loop fasteners 41 and 51, whereby the belt 1 can keep its shape even in water. Herein the position of the annular member 50 on the belt 1 can be changed.

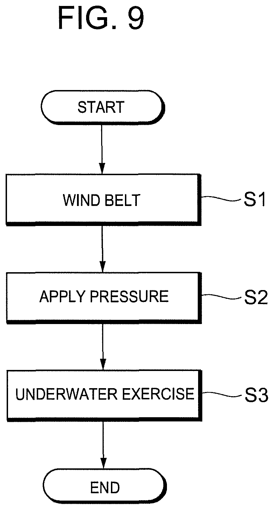

Next, the following describes each step of the KAATSU underwater training, referring to the flowchart of FIG. 9 and the like.

Firstly, the belt 1 is wound around at least one (e.g., left upper arm including biceps) of four limbs of the user including a muscle to be strengthened at a part that is closer to the heart so as to form a loop shape (belt winding step: S1). At this time, the belt-like member 40 and the other end 5 of the belt 1 are passed through the buckle 20 attached at the one end 4 of the belt 1 and then the belt 1 is folded back.

Next, the belt 1 is tightened while holding the belt-like member 40 passing through the buckle 20, whereby the loop shape of the belt 1 is kept using the hook-and-loop fastener 40 while restricting the blood circulation of the muscle of the user by applying specific pressure to the muscle of the user (pressure applying step: S2). The magnitude of the specific pressure can be set appropriately depending on the age, gender, exercise experiences or the like of the user.

Subsequently, the user is asked to perform underwater exercise while keeping the loop shape of the belt 1 (while keeping the specific pressure applied to the muscle from the belt 1 constant) (underwater exercise step: S3). In the underwater exercise step S3, a resisting force or a buoyancy force of water will act on the user. Types or the duration of the underwater exercise can be appropriately set depending on the age, gender, exercise experiences or the like of the user.

In the muscle training method according to the thus described embodiment, the belt 1 is wound around at least one of four limbs of a user to apply specific pressure to a muscle of the user to restrict the blood circulation there without stopping it, whereby growth hormones of the user can be produced more. Further, a user who conducts such underwater exercise while restricting the blood circulation appropriately can receive a load that cannot be received on the ground. That is, the blood circulation of the muscle of the user is appropriately restricted, whereby the level of lactic acid in the blood of the user can be increased to produce growth hormones more without the need of employing a large tool or such equipment as in the conventional underwater training method. Additionally a load can be given effectively from motion less than on the ground, and from the synergic action of them, muscles can be strengthened very easily. Moreover, since a buoyancy force also can be obtained in water, a user who has difficulty in supporting their weight because of muscle strength declined also can strengthen their muscles using this method.

### <Example>

Next, the following describes an example of the present invention.

### <Pre-test>

In this example, firstly each of sixteen examinees aged between 12 and 14 was asked to perform pre-test. In the pre-test, each examinee was asked to swim the distance of 50 yards with the maximum force within 1 minute, followed by interval for 15 seconds, and they were asked to swim the distance of 50 yards with the maximum force within 1 minute again (in other words, they started the next swim after 1 minute and 15 seconds from the starting time of the preceding swim regardless of the time required for the 50-yard swim), and to repeat this interval exercise 10 times.

### <KAATSU underwater training>

After the pre-test, the sixteen examinees were divided into an "experiment group" consisting of eight examinees performing the KAATSU underwater training, and a "control group" consisting of eight examinees not performing the KAATSU underwater training, and then the eight examinees in the "experiment group" were asked to perform the following muscle training for 11 days.

### <The first day through the third day>

From the first day to the third day, the following training was performed. Firstly the belt 1 as described in the above embodiment was wound around the base of an arm of each of the eight examinees in the "experiment group" to apply specific pressure to restrict the blood circulation of the muscle of the examinee's arm without stopping it for pressurization to the arm. At this time, the specific pressure was set at "80 mmHg". Then, while conducting such pressurization to the arm, the examinees were asked to conduct interval exercise for 15 minutes that was similar to that performed in the pre-test, and thereafter the belt 1 wound around the arm of the examinees was removed to cancel the pressurization to the arm.

Next, the belt 1 was wound around the base of a leg of each of the eight examinees in the "experiment group" to apply specific pressure to restrict the blood circulation of the muscle of the examinee's leg of the examinee without stopping it for pressurization to the leg. At this time, the specific pressure was set at "100 mmHg". Then, while conducting such pressurization to the leg, the examinees were asked to conduct interval exercise for 15 minutes that was similar to that performed in the pre-test, and thereafter the belt 1 wound around the leg of the examinees was removed to cancel the pressurization to the leg.

In this way, from the first day to the third day, the interval exercise for 15 minutes involving the pressurization to an arm at "80 mmHg" and the interval exercise for 15 minutes involving the pressurization to a leg at "100 mmHg" were performed. Herein, the specific pressure "80 mmHg" and "100 mmHg" were values set to be relatively low for the purpose of letting the examinees get accustomed to the pressurization to arm and leg.

### <The fourth day through the tenth day>

From the fourth day to the tenth day, the following training was performed. Firstly the belt 1 was wound around the base of an arm of each of the eight examinees in the "experiment group" to apply specific pressure to restrict the blood circulation of the muscle of the examinee's arm without stopping it for pressurization to the arm. At this time, the specific pressure was set at "120 mmHg". Then, while conducting such pressurization to the arm, the examinees were asked to conduct specific interval exercise (specific swim exercise), and thereafter the belt 1 wound around the arm of the examinees was removed to cancel the pressurization to the arm.

The specific interval exercise (specific swim exercise) involving the pressurization to the arm from the fourth day to the tenth day includes: (1) a first step of performing arm/leg propulsion exercise of 200 yards continuously to move forward by making strokes with the arms and by kicking water; (2) a second step of performing the arm/leg propulsion exercise of 50 yards four times while having a specific stop duration therebetween; and (3) a third step of performing arm propulsion exercise of 50 yards to move forward by making strokes with the arms only, followed by the arm/leg propulsion exercise of 50 yards while having a specific stop duration therebetween. At this time, the arm/leg propulsion exercise in the first step was performed with relatively mild intensity (e.g., about 50%), the arm/leg propulsion exercise in the second step was performed with the intensity of about 85%, and the arm/leg propulsion exercise in the third step was performed with the intensity of 100%. That is, the arm/leg propulsion exercise in the third step was performed with intensity higher than that of the arm/leg propulsion exercise in the second step, and the arm/leg propulsion exercise in the second step was performed with intensity higher than that of the arm/leg propulsion exercise in the first step.

Next the belt 1 was wound around the base of a leg of each of the eight examinees in the "experiment group" to apply specific pressure to restrict the blood circulation of the muscle of the examinee's leg without stopping it for pressurization to the leg. At this time, the specific pressure was set at "140 mmHg". Then, while conducting such pressurization to the leg, the examinees were asked to conduct specific interval exercise (specific swim exercise), and thereafter the belt 1 wound around the leg of the examinees was removed to cancel the pressurization to the leg.

The specific interval exercise (specific swim exercise) involving the pressurization to the leg from the fourth day to the tenth day includes: (1) a sixth step of performing the arm/leg propulsion exercise of 25 yards continuously four times; and (2) a seventh step of performing the leg propulsion exercise of 50 yards to move forward by kicking water only, followed by the arm/leg propulsion exercise of 50 yards while having a specific stop duration therebetween. In this example, the seventh step was repeated twice, and the arm/leg propulsion exercise in the sixth step and the arm/leg propulsion exercise in the seventh step were performed with the intensity of 100%.

In this way, from the fourth day to the tenth day, the specific interval exercise involving the pressurization to an arm at "120 mmHg" and the specific interval exercise involving the pressurization to a leg at "140 mmHg" were performed.

### <The eleventh day>

On the eleventh day, the following training was performed. Firstly the belt 1 was wound around the base of an arm of each of the eight examinees in the "experiment group" to apply specific pressure to restrict the blood circulation of the muscle of the examinee's arm without stopping it for pressurization to the arm. At this time, the specific pressure was set at "120 mmHg". Then, while conducting such pressurization to the arm, the examinees were asked to conduct specific interval exercise (specific swim exercise), and thereafter the belt 1 wound around the arm of the examinees was removed to cancel the pressurization to the arm.

The specific interval exercise (specific swim exercise) involving the pressurization to the arm on the eleventh day includes: (1) a first step of performing the arm/leg propulsion exercise of 200 yards continuously; (2) a fourth step of performing the arm propulsion exercise of 75 yards, followed by the arm/leg propulsion exercise of 75 yards; and (3) a fifth step of performing the arm/leg propulsion exercise of 25 yards four times while having a specific stop duration therebetween. At this time, the arm/leg propulsion exercise in the first step was performed with relatively mild intensity (e.g., about 50%), the arm/leg propulsion exercise in the fourth step was performed with the intensity of 100%, and the arm/leg propulsion exercise in the fifth step was performed with the intensity of 100%. That is, the arm/leg propulsion exercise in the fifth step was performed with intensity equal to the intensity of the arm/leg propulsion exercise in the fourth step, and the arm/leg propulsion exercise in the fourth and the fifth steps was performed with intensity higher than that of the arm/leg propulsion exercise in the first step.

Next the belt 1 was wound around the base of a leg of each of the eight examinees in the "experiment group" to apply specific pressure to restrict the blood circulation of the muscle of the examinee's leg without stopping it for pressurization to the leg. At this time, the specific pressure was set at "140 mmHg". Then, while conducting such pressurization to the leg, the examinees were asked to conduct specific interval exercise (specific swim exercise), and thereafter the belt 1 wound around the leg of the examinees was removed to cancel the pressurization to the leg.

The specific interval exercise (specific swim exercise) involving the pressurization to the leg on the eleventh day includes: (1) an eighth step of performing the leg propulsion exercise of 75 yards, followed by the arm/leg propulsion exercise of 75 yards while having a specific stop duration therebetween; and (2) a ninth step of performing the arm/leg propulsion exercise of 25 yards four times while having a specific stop duration therebetween. In this example, the arm/leg propulsion exercise in the eighth step and the arm/leg propulsion exercise in the ninth step were performed with the intensity of 100%.

In this way, on the eleventh day, the specific interval exercise involving the pressurization to an arm at "120 mmHg" (interval exercise different from that performed from the fourth day to the tenth day) and the specific interval exercise (interval exercise different from that performed from the fourth day to the tenth day) involving the pressurization to a leg at "140 mmHg" were performed.

### <Post-test>

Subsequently, each of the eight examinees in the "experiment group" who conducted the KAATSU underwater training was asked to perform post-test similar to the pre-test. The eight examinees in the "control group" who did not conduct the KAATSU underwater training also were asked to perform similar post-test.

### <Test result>

In the result of measuring the time (average of ten times) required for swimming of 50 yards of the eight examinees in the "experiment group" who conducted the KAATSU underwater training, while the average of the eight examinees in the pre-test was 33.968 seconds, the average of the eight examinees in the post-test was 32.381 seconds, and the difference therebetween was about 1.59 seconds. This means that the required time was shortened by up to 4.671%.

On the contrary, in the result of measuring the time (average of ten times) required for swimming of 50 yards of the eight examinees in the "control group" who did not conduct the KAATSU underwater training, while the average of the eight examinees in the pre-test was 33.9 seconds, the average of the eight examinees in the post-test was 33.778 seconds, and the difference therebetween was just 0.122 second. This means that the required time was shortened by just 0.36%.

In this way, the eight examinees in the "experiment group" who conducted the KAATSU underwater training showed significant improvement of their muscle strengths.

Note here that the "underwater exercise" in the present invention is not limited to the swim exercise described in the above example, which may include other exercises in water (walking exercise, squat exercise, flutter-kick exercise or kick exercise while holding the poolside, calf raise (standing on tiptoe) exercise, leg curl exercise, leg extension exercise and the like). The swim exercise in the above example shows an example, in which the pressure applied to an arm was set at 80 to 120 mmHg, and the pressure applied to a leg was set at 100 to 140 mmHg. When an exercise that is milder than the swim exercise (e.g., walking exercise in water) is to be performed, the pressure applied to an arm may be set at 120 mmHg or more, and the pressure applied to a leg may be set at 140 mmHg or more.

Recent advanced study on the KAATSU training method shows that growth hormones are produced more than usual from the pituitary gland through appropriate pressurization and that these growth hormones have a favorable influence on the body in addition to strengthening of muscles. For this reason, the KAATSU training method is now increasingly applied to the field of medicine or rehabilitation. The muscle training method according to the present invention is the combination of this "KAATSU" with "underwater exercise", which can promote the production of growth hormones more, and therefore such a method can be applied well to the field of medicine or rehabilitation as well, and can realize prevention or treatment of various types of diseases (diabetes, high blood pressure, hyperlipidemia and the like).

The present invention is not limited to the embodiments as stated above, and design modifications to these embodiments, which will be made by a person skilled in the art as appropriate, are also included in the scope of the present invention as long as they have the features of the present invention. That is, each element in the above specific examples and the arrangement, materials, conditions, shapes, dimensions, etc., thereof are not limited to those described above and may be modified as appropriate. Each element in these embodiments can be combined as long as such combination is technically possible, and such a combination also is included in the scope of the present invention as long as they have the features of the present invention.

**Reference Numerals** 1 belt

## Claims

1. A muscle training method for training of a muscle of a user, comprising: asking the user to perform underwater exercise while winding a belt around at least one of four limbs of the user and applying specific pressure to restrict blood circulation at a muscle of the user without stopping the blood circulation.

2. The muscle training method according to claim 1, wherein the belt is wound around a base of an arm of the user and specific pressure is applied thereto to restrict blood circulation at a muscle of the arm of the user without stopping the blood circulation for arm pressurization.

3. The muscle training method according to claim 2, wherein the specific pressure is set in a range of 80 to 120 mmHg.

4. The muscle training method according to claim 3, wherein while receiving the arm pressurization, the user is asked to perform specific swim exercise including an arm/leg propulsion exercise to move forward by making strokes with arms and by kicking water, and an arm propulsion exercise to move forward by making strokes with the arms only.

5. The muscle training method according to claim 4, wherein the specific swim exercise includes: a first step of performing the arm/leg propulsion exercise of 200 yards continuously; a second step of performing the arm/leg propulsion exercise of 50 yards four times while having a specific stop duration therebetween; and a third step of performing the arm propulsion exercise of 50 yards, followed by the arm/leg propulsion exercise of 50 yards while having a specific stop duration therebetween.

6. The muscle training method according to claim 5, wherein the arm/leg propulsion exercise in the second step is performed with intensity higher than in the arm/leg propulsion exercise in the first step.

7. The muscle training method according to claim 5 or 6, wherein the arm/leg propulsion exercise in the third step is performed with intensity higher than in the arm/leg propulsion exercise in the second step.

8. The muscle training method according to claim 4, wherein the specific swim exercise includes: a first step of performing the arm/leg propulsion exercise of 200 yards continuously; a fourth step of performing the arm propulsion exercise of 75 yards, followed by the arm/leg propulsion exercise of 75 yards; and a fifth step of performing the arm/leg propulsion exercise of 25 yards four times while having a specific stop duration therebetween.

9. The muscle training method according to claim 8, wherein the arm/leg propulsion exercise in the fourth step is performed with intensity higher than in the arm/leg propulsion exercise in the first step.

10. The muscle training method according to claim 8 or 9, wherein the arm/leg propulsion exercise in the fifth step is performed with intensity equal to intensity of the arm/leg propulsion exercise in the fourth step.

11. The muscle training method according to claim 2, wherein the user is asked to perform walking exercise in water while setting the specific pressure for the arm pressurization at a value exceeding 120 mmHg.

12. The muscle training method according to claim 1, wherein the belt is wound around a base of a leg of the user and specific pressure is applied thereto to restrict blood circulation at a muscle of the leg of the user without stopping the blood circulation for leg pressurization.

13. The muscle training method according to claim 12, wherein the specific pressure is set in a range of 100 to 140 mmHg.

14. The muscle training method according to claim 13, wherein while receiving the leg pressurization, the user is asked to perform specific swim exercise including an arm/leg propulsion exercise to move forward by making strokes with arms and by kicking water, and a leg propulsion exercise to move forward by kicking water only.

15. The muscle training method according to claim 14, wherein the specific swim exercise includes: a sixth step of performing the arm/leg propulsion exercise of 25 yards four times while having a specific stop duration therebetween; and a seventh step of performing the leg propulsion exercise of 50 yards, followed by the arm/leg propulsion exercise of 50 yards while having a specific stop duration therebetween.

16. The muscle training method according to claim 15, wherein the seventh step is repeated twice.

17. The muscle training method according to claim 14, wherein the specific swim exercise includes: an eighth step of performing the leg propulsion exercise of 75 yards, followed by the arm/leg propulsion exercise of 75 yards while having a specific stop duration therebetween; and a ninth step of performing the arm/leg propulsion exercise of 25 yards four times while having a specific stop duration therebetween.

18. The muscle training method according to claim 12, wherein the user is asked to perform walking exercise in water while setting the specific pressure for the leg pressurization at a value exceeding 140 mmHg.

19. The muscle training method according to claim 1, comprising: winding the belt around a base of an arm of the user and applying specific pressure thereto to restrict blood circulation at a muscle of the arm of the user without stopping the blood circulation for arm pressurization; and winding the belt around a base of a leg of the user and applying specific pressure thereto to restrict blood circulation at a muscle of the leg of the user without stopping the blood circulation for leg pressurization, which is performed after the arm pressurization.

20. The muscle training method according to claim 19, wherein the specific pressure for the arm pressurization is set in a range of 80 to 120 mmHg, and the specific pressure for the leg pressurization is set in a range of 100 to 140 mmHg.

21. The muscle training method according to claim 19 or 20, wherein
while receiving the arm pressurization, the user is asked to perform specific swim exercise including an arm/leg propulsion exercise to move forward by making strokes with arms and by kicking water, and an arm propulsion exercise to move forward by making strokes with the arms only, and
while receiving the leg pressurization, the user is asked to perform specific swim exercise including the arm/leg propulsion exercise, and a leg propulsion exercise to move forward by kicking water only.

22. The muscle training method according to claim 19, wherein while the specific pressure for the arm pressurization is set at a value exceeding 120 mmHg and the specific pressure for the leg pressurization is set at a value exceeding 140 mmHg, the user is asked to perform walking exercise in water.
